# EUROPEAN PATENT SPECIFICATION

(11) **EP 1 491 149 B1**
(45) Date of publication and mention of the grant of the patent: **09.05.2007**
(21) Application number: 04253365.3
(22) Date of filing: 04.06.2004
(51) Int. Cl.: A61B 17/16

(54) **Non-linear reamer for bone preparation**
Gelenkfräsvorrichtung
Aléseur articulé

(30) Priority: 25.06.2003 US 606304
(43) Date of publication of application: 29.12.2004
(60) Divisional application: 06024884.6
(73) Proprietor: DePuy Products, Inc., Warsaw, Indiana 46581 (US)
(72) Inventor: Daniels, David Wayne, Warsaw IN 46582 (US); Dwyer, Kimberly Ann, Ft Wayne IN 46818 (US)
(74) Representative: Belcher, Simon James

(56) References cited:
- EP-A- 1 201 191
- US-A- 5 957 925
- US-A- 6 117 138
- US-A1- 2001 016 779

## Description

The present invention relates generally to the field of orthopaedics, and more particularly, to an implant for use in arthroplasty.

Patients who suffer from the pain and immobility caused by osteoarthritis and rheumatoid arthritis have an option of joint replacement surgery. Joint replacement surgery is quite common and enables many individuals to function properly when it would not be otherwise possible to do so. Artificial joints are usually comprised of metal, ceramic and/or plastic components that are fixed to existing bone.

Such joint replacement surgery is otherwise known as joint arthroplasty. Joint arthroplasty is a well-known surgical procedure by which a diseased and/or damaged joint is replaced with a prosthetic joint. In a typical total joint arthroplasty, the ends or distal portions of the bones adjacent to the joint are resected or a portion of the distal part of the bone is removed and the artificial joint is secured thereto.

There are known to exist many designs and methods for manufacturing implantable articles, such as bone prostheses. Such bone prostheses include components of artificial joints such as elbows, hips, knees and shoulders.

Currently in total hip arthroplasty, a major critical concern is the instability of the joint. Instability is associated with dislocation. Dislocation is particularly a problem in total hip arthroplasty.

Factors related to dislocation include surgical technique, implant design, implant positioning and patient related factors. In total hip arthroplasty, implant systems address this concern by offering a series of products with a range of lateral offsets, neck offsets, head offsets and leg lengths. The combination of these four factors affects the laxity of the soft tissue. By optimizing the biomechanics, the surgeon can provide a patient a stable hip much more resistant to dislocation.

In order to accommodate the range of patient arthropometrics, a wide range of hip implant geometries are currently manufactured by DePuy Orthopaedics Inc and by other companies. In particular, the total hip systems sold by DePuy Orthopaedics Inc under the trade mark S-ROM include three offsets, three neck lengths, four head lengths and one leg length adjustment. The combination of all these biomechanic options is rather complex.

Anteversion of a total hip system is closely linked to the stability of the joint. Improper anteversion can lead to dislocation and patient dissatisfaction. Anteversion control is important in all hip stems. However, it is a more challenging issue with the advent of stems with additional modularity.

The prior art has provided for some addressing of the anteversion problem. For example, currently available stems have laser markings on the medial stem and the proximal sleeve. This marking enables the surgeon to measure relative alignment between these components. Since the sleeve has infinite anteversion, it is not necessarily oriented relative to a bony landmark that can be used to define anteversion. In fact, the current sleeves are sometimes oriented with the spout pointing directly laterally into the remaining available bone.

When a primary or index total joint arthroplasty fails, a revision procedure is performed in which the index devices (some or all) are removed. Quite often the remaining bone is significantly compromised compared to a primary hip procedure. Significant bone loss is observed, often with a lack of bone landmarks typically used for alignment.

Prior art stems may be aligned relative to a patient's bony landmarks. These stems are monolithic. They cannot locate the neck independently of the distal stem. Therefore, the anteversion is limited. Most bowed, monolithic stems are sold in fixed anteversion; for example, at an anteversion of 15°. These monolithic stems have limited flexibility for rotational alignment since the distal stem must follow the bow of the patient's femur and this may not provide an operable biomechanical result.

In a common step in the surgical procedure known as total hip arthroplasty, a trial or substitute stem is first implanted into the patient. The trial is utilized to verify the selected size and shape of the implant *in situ* on the patient and the patient is subjected to what is known as a trial reduction. This trial reduction represents moving the joint, including the trial implant through selected typical motions for that joint. Current hip instruments provide a series of trials of different sizes to help the surgeon assess the fit and position of the implant. Trials, which are also known as provisionals, allow the surgeon to perform a trial reduction to assess the suitability of the implant and the implant's stability prior to final implant selection. In order to reduce inventory costs and complexity, many trialing systems are modular. For example, in the instrument system sold by DePuy Orthopaedics Inc under the trade mark Excel, there is a series of broaches and a series of neck trials that can be mixed and matched to represent the full range of implants. There is a single fixed relationship between a broach and a neck trial, because these trials represent a system of monolithic stem implants.

Likewise, in instrument systems sold by DePuy Orthopaedics Inc under the trade mark S-ROM, there are neck trials, proximal body trials, distal stem trials, head trials and sleeve trials. By combining all of these components, the implant is represented. Since the implant is modular and includes a stem and a sleeve, the angular relationship or relative anteversion between the neck and the sleeve is independent and represented by teeth mating between the neck and the proximal body trial. The proximal body trial has fixed transverse bolts that are keyed to the sleeve in the trialing for straight, primary stems. The long stem trials do not have the transverse bolts and are thus not rotationally stable during trial reduction and therefore are not always used by the surgeon.

With the introduction of additional implant modularity, the need for independent positioning of the distal stem, proximal body and any sleeve that comprise the implants is required. Currently bowed, monolithic stems are offered with a fixed amount of anteversion, typically 15°.

Many problems exist with the current instruments, implants and surgical procedures in joint replacement. One of these problems is the improper alignment of the prosthesis in the bone. The improper alignment provides for increased likelihood of dislocation of the joint and for sub-optimizing the soft tissue tension resulting in, for example, laxity of the soft tissue. This laxity can further lead to dislocation. The improper alignment also affects the patient's satisfaction. Improper alignment may also result in improper anteversion and resulting problems with patient satisfaction.

Currently available implants, trials and instruments result in a lengthy surgical procedure. This lengthy surgical procedure includes the steps of preparing the canal, removing the instruments to prepare the canal, implanting trials, performing a trial reduction and then implanting the prosthesis. This lengthy procedure increases the risk of the patient's surgical complications.

When utilizing currently available instruments, trials and surgical procedures, the surgeon must perform the trial reduction on the patient before the surgeon has any feedback regarding the appropriateness of the trial and the positioning of the trial in the body. Adjustments in the positioning and selection of the trial and resultant implants thus become difficult and time consuming to perform.

Utilizing the current instruments, the trials and implants all need to be properly located and selected to obtain the optimum results for the patient. The positioning of the trial with respect to the femur and the implant with respect to the trial currently allow for much variation from procedure to procedure.

To optimize patient outcomes, orthopaedic surgery preferably conserves as much of the resected bone as possible. Current surgical procedures require that sufficient bone be resected and removed by instruments in the proximal bone to provide for clearance for the proximal trial and the proximal implant. Thus, under current techniques, material must be removed proximally on the bone to provide for the variety of positions that may be optimum for the patient.

US 5,957,925 discloses a reaming system comprising a shaft on which a proximal reamer is permanently fixed, the distal end of the shaft having a hinge portion. A distal reamer can be connected to the distal end of the hinge portion by means of a keying arrangement such that the shaft can transmit rotational motion to the distal reamer.

EP-A-1201191 discloses a reaming system which makes use of a shaft which can be flexed to enable the axis of the proximal portion of the shaft to be varied relative to the axis of the distal portion of the shaft.

Reference is made to EP-A-1 430 859 which discloses an adjustable biomechanical templating & resection instrument and an associated method, and to EP-A-1 437 107 which discloses an alignment device for modular implants and an associated method, and to EP-A-1 435 223 which discloses an instrument and an associated method of trialing for modular hip stems.

The present invention allows for an accurate measurement of hip biomechanics in reference to the implant system without the use of a distal trial. The present invention provides for a reamer that replicates the implant configuration for a long anatomically curved distal stem. The articulating reamer provides centralization and proper alignment for a proximal reamer. The articulating reamer provides for correct orientation for the trialing for the hip reduction. By trailing off the reamer, the need for distal stem trials is eliminated. The articulating reamer has a first position that is straight in which the reamer is used to prepare the bone canal. The reamer also has a second position in which a first portion and a second portion of the reamer are skewed. After the reamer has been used to prepare the canal, the reamer is skewed and locked in the skewed position. A proximal trial is placed over the proximal portion of the reamer and is used to perform the trial reduction on the patient. The orientation of the skewed reamer may be measured by a tool that can be used to replicate a corresponding orientation of the appropriate implant.

Accordingly, in one aspect, the invention provides a reamer for preparing a cavity in the intramedullary canal of a long bone, which comprises:
a distal component for preparation of the cavity in the canal, the distal component including a first reamer portion thereof for placement at least partially in the cavity of the long bone, the distal component defining a rotational centerline thereof,
a proximal component on which a second reamer portion or a trial component can be mounted, which is connected to the distal component by means of a hinge, the proximal component defining a rotational centerline thereof, the rotational centerline of the distal component and the rotational centerline of the proximal component having a first relationship in which the centerlines are coincident and a second relationship in which the centerlines are skewed with respect to each other, and
a locking arrangement by which the angular relationship between the distal and proximal components can be locked against movement from the second relationship towards the first relationship.

In another aspect, the invention provides a reamer assembly for preparing a cavity in the intramedullary canal of a long bone. The reamer assembly includes a reamer as discussed above, and a second reamer portion which can be fitted over at least a portion of second component by sliding.

In yet another aspect, the invention provides a kit for preparing a cavity in the intramedullary canal of a long bone for use in performing joint arthroplasty, which comprises a reamer as discussed above, and a trial for assisting in performing a trial reduction which can be fitted to the reamer.

The instruments of the invention can be used in a method for providing joint arthroplasty which includes the steps of opening a medullary canal of the long bone, providing a reamer including a first member having a first member centerline and a second member having a second member centerline, the first member centerline being movable with respect to the second member centerline, the first member including a surface for the removal of bone, positioning the reamer in the canal, reaming a cavity in the canal with the reamer with the first member centerline being coincident with the second member centerline, and adjusting the reamer such that the first member centerline is skewed with respect to the second member centerline.

The technical advantages of the present invention include the non-linear shape of the reamer that replicates the implant configuration. For example, according to one aspect of the present invention, the reamer of the present invention includes a first portion and a second portion that articulates with respect to the first portion. The angle configuration of the reamer thereby replicates the distal stem of an implant that is bowed. Thus the present invention provides for a reamer shape that replicates the implant configuration.

The technical advantages of the present invention also include a reduction in surgical time. The reduction in surgery time improves patient outcomes and reduces the complications caused by extended surgery. For example, according to one aspect of the present invention, a surgical procedure is provided which includes the placing of a proximal trial on a reamer positioned in the femoral canal. Trialing thus occurs off the reamer and eliminates the need to remove the reamer and place a distal stem trial into the femoral canal. Thus, the present invention provides for an eliminated step and a consequential reduced surgery time.

The technical advantages of the present invention further include improving the biomechanics of the resultant implant. The improved biomechanics are the result of an improved positioning of the implant that also optimizes the soft tissue tension and eliminates soft tissue laxity. For example, according to one aspect of the present invention, a proximal trial may be placed on a reamer still in place on the femoral canal and a trial reduction performed. The configuration of the trial reamer assembly may be duplicated in a modular prosthesis that will be properly positioned in the canal based upon the optimum position obtained during the trialing. Thus, the present invention provides for improved biomechanics.

Yet another technical advantage of the present invention is an improvement in the anteversion resulting from the implant. Optimum anteversion or the angle for the location of the joint in the body serves to reduce dislocation. For example, according to one aspect of the present invention, the reamer includes a proximal portion and a distal portion that is pivotally positioned with respect to the distal portion.

While the reamer is straight, the cavity is prepared including a portion of the curved portion of the canal. The reamer is then pivoted to obtain a shape similar to that of a bowed distal trial. A proximal trial is placed upon the reamer and a trial reduction is performed. Thus a position of the non-linear reamer with respect to the proximal body of the trial may be duplicated onto an implant. The distal stem of the implant is thus positioned into the bow of the femur. The implant thus duplicates the anteversion of the reamer trial assembly. The bowed distal stem reamer is aligned rotationally in the intramedullary canal. The proximal trial is positioned on the distal reamer and is rotated until proper anteversion is achieved. This position can be locked in and measured with auxiliary instruments and reproduced in the final implants. Thus, the present invention provides for improved anteversion.

A further technical advantage of the present invention is the reduction of inventory and the simplification of the instrumentation for the operating room staff. By providing a reamer that may receive a proximal trial, the need for distal trial stems is eliminated. For example, according to one aspect of the present invention, a proximal trial is operably connected to a reamer while the reamer is still in position in the canal. The trial reduction may be performed with the combination of a reamer and proximal trial. The need for an additional stem trial is therefore eliminated. The elimination of the distal stem trial reduces the need for inventory or the manufacturing capability, tooling and inventory necessary for the distal stem trials. Thus, the present invention provides for reduced inventory. Further, the elimination of the need for distal stem trials reduces the complexity of the instruments in the operating room, simplifying the operating room procedures.

Yet another technical advantage of the present invention is the ability to provide immediate feedback on the leg length prior to trial reduction. For example, according to one aspect of the present invention, the reamer and driver assembly may include marks or indicia on the assembly used to measure by sight the position of the reamer relative to a landmark on the hip. Such measurement is related to leg length. Thus, the present invention provides for immediate feedback on the leg length prior to trial reduction.

Yet another technical advantage of the present invention includes the ability of the alignment of the reamer, trial and implant to be timed, matched and duplicated. For example, according to one aspect of the present invention, the reamer, trial and implant each have orientation marks or reference points which may measure and align the proximal trial to the reamer or the proximal implant to the distal stem implant. Thus the present invention provides for timing, matching and duplication of the alignment of the reamer, trial and implant.

Yet another technical advantage of the present invention includes minimizing of bone removal in the proximal portion of the long bone. Minimal bone removal is generally referred to as bone preservation and is highly favoured by surgeons to improve patient outcomes. For example, according to one aspect of the present invention, the distal reamer includes a portion for receiving a proximal reamer. The proximal reamer is thus guided by the distal reamer to be sure that the proximal reaming occurs at the optimum location corresponding to where the proximal trial and proximal implant body will preferably be located. By providing the proximal reaming in the anatomically correct position, the reaming of bone to provide clearance for the trial and implant can be minimized, thus minimizing the bone removal in the proximal area of the long bone. Thus, the present invention provides for minimal bone removal.

Embodiments of the invention will now be described by way of example with reference to the accompanying drawings, in which:
FIG. 1 is a plan view of a non-linear reamer assembly including proximal and distal reaming portions in the articulated position in accordance with an embodiment of the present invention;
FIG. 2 is a bottom view of the distal reamer of the reamer assembly of FIG. 1;
FIG. 3 is a partial plan view of the distal reamer of the reamer assembly of FIG. 1 showing the proximal end in greater detail;
FIG. 4 is a plan view of the reamer assembly of FIG. 1 including the reamer driver;
FIG. 5 is an exploded perspective view of the reamer assembly of FIG. 1 including the reamer driver;
FIG. 6 is a cross sectional view of the reamer driver of the reamer assembly FIG. 5 along the line 6-6 in the direction of the arrows;
FIG. 7 is a plan view of a proximal reamer for use with the distal reamer of the reamer assembly of FIG. 1;
FIG. 8 is a plan view of the distal reamer of the reamer assembly of FIG. 1 with the reamer in a flaccid condition;
FIG. 9 is a partial plan view of the distal reamer of FIG. 8 showing the pivot point in greater detail;
FIG. 10 is a partial plan view of the distal reamer of FIG. 8 showing the pivot point in greater detail with the view rotated 90° from that of FIG. 9;

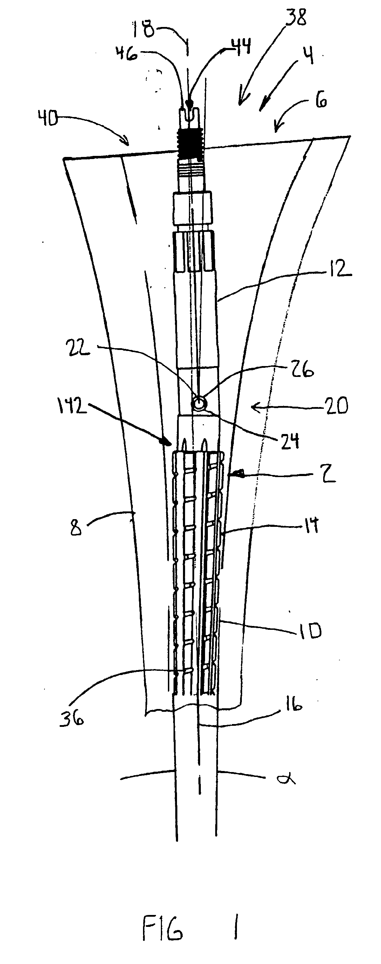

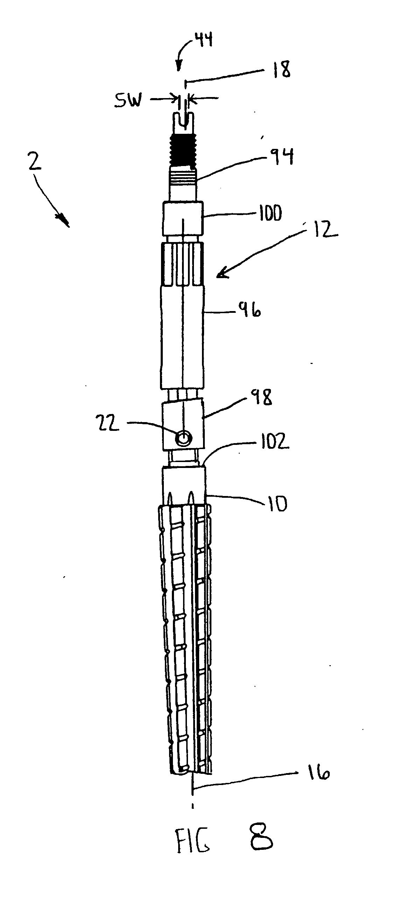

FIG. 1 is an end view of a hollow cylindrical wedge for use with the distal reamer of FIG. 8;

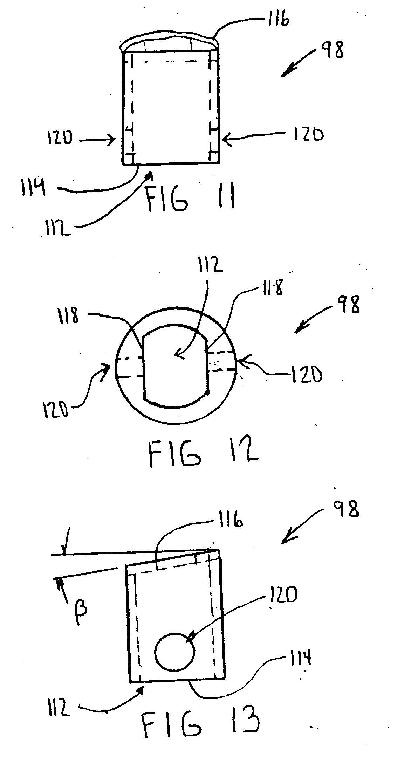

FIG. 12 is a top view of the hollow cylindrical wedge of FIG. 11;
FIG. 13 is a plan view of the hollow cylindrical wedge of FIG. 11;

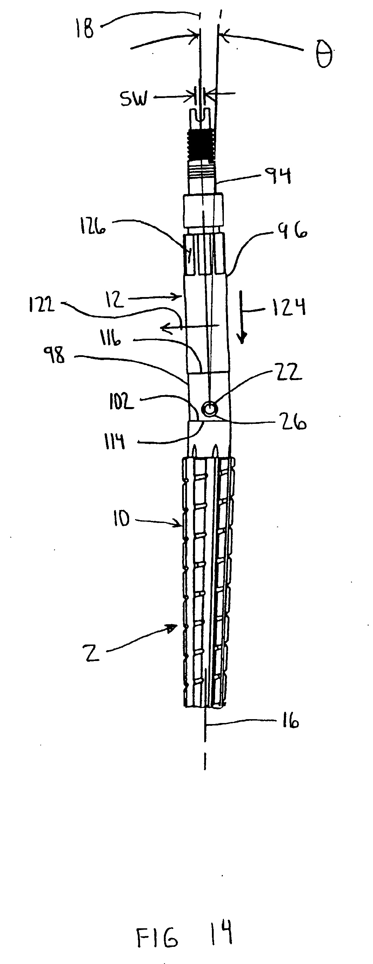

FIG. 14 is a plan view of the reamer of the reamer assembly of FIG. 1 showing the reamer in an articulated position;

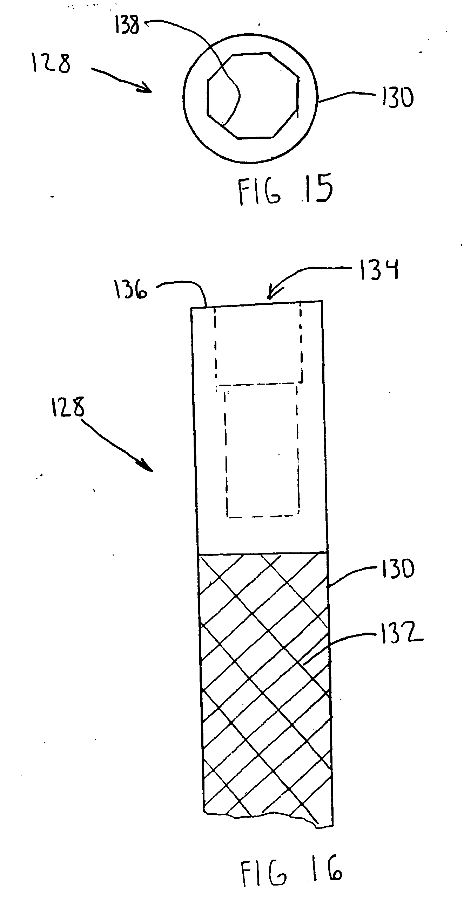

FIG. 15 is a top view of a wrench for use with the reamer of the reamer assembly of FIG. 1;

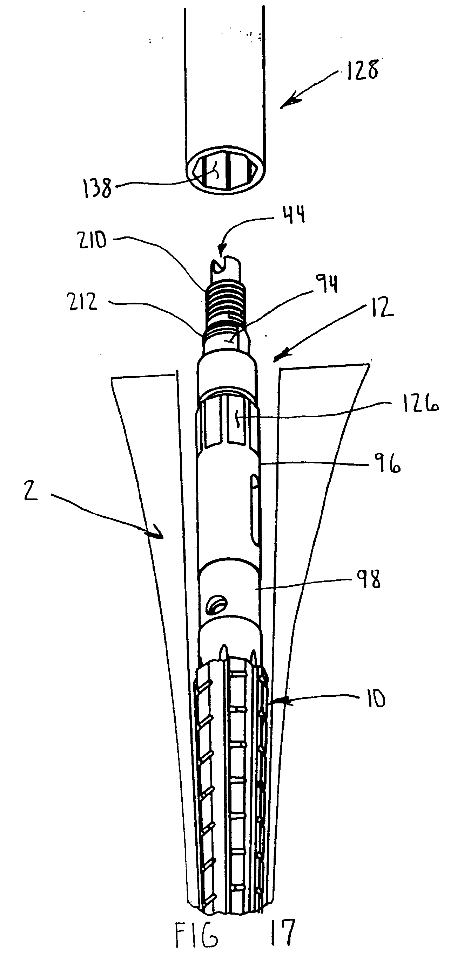

FIG. 16 is a plan view of the wrench of FIG. 15;

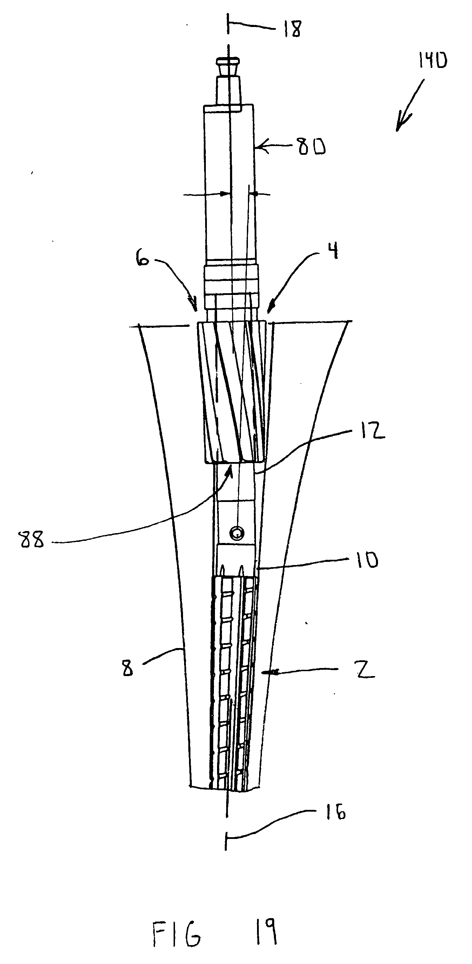

FIG. 17 is a partial exploded perspective view of the reamer assembly of FIG. 1 including the reamer driver of FIG. 19;

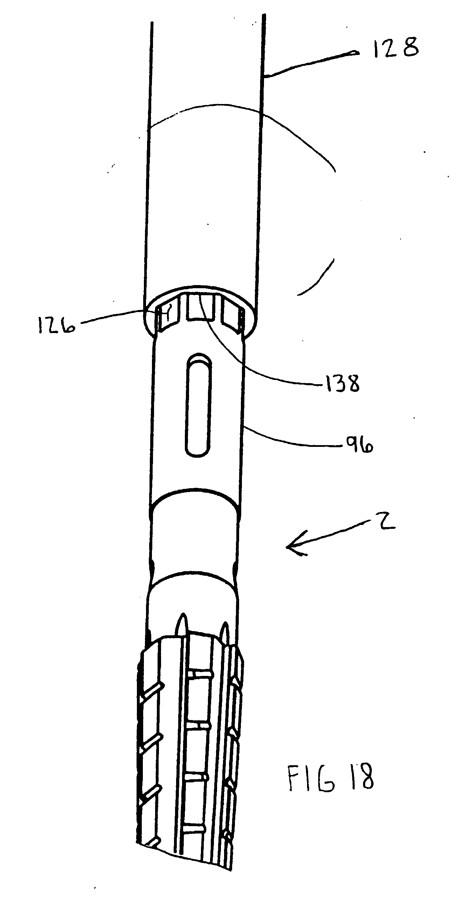

FIG. 18 is a partial perspective view of the reamer assembly of FIG. 1 including the reamer driver;

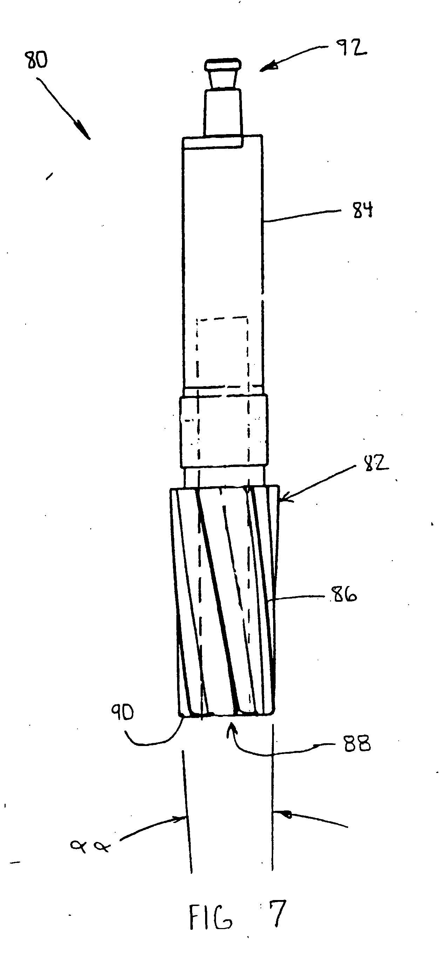

FIG. 19 is an exploded perspective view of the reamer assembly of FIG. 1 including the proximal reamer of FIG. 7 in accordance with an embodiment of the present invention;

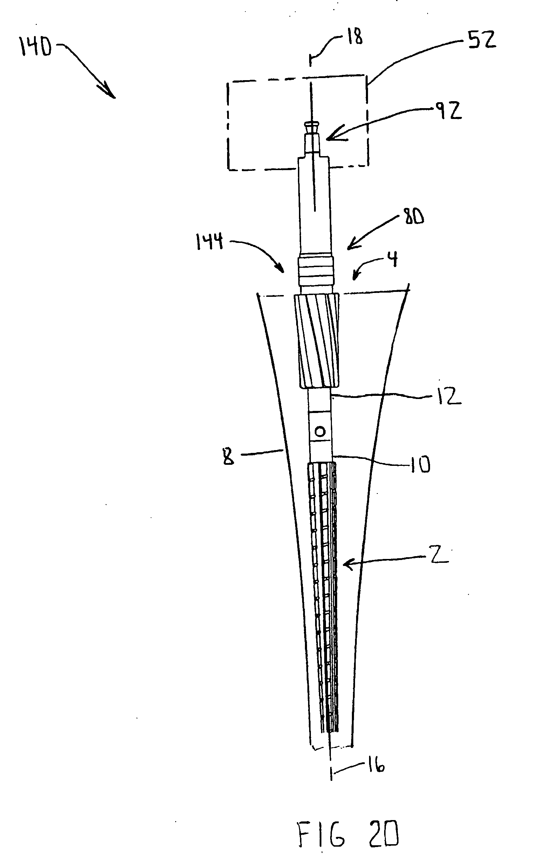

FIG. 20 is a plan view of the reamer assembly of FIG. 1 including proximal and distal reaming portions in the straight position;

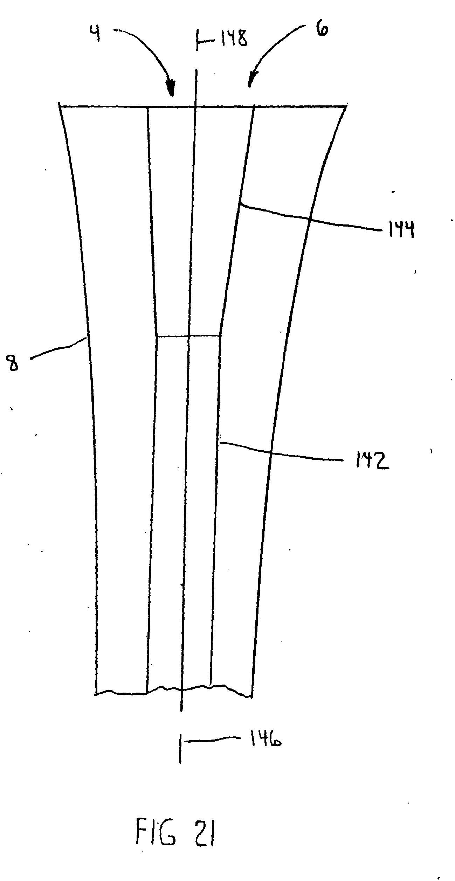

FIG. 21 is a cross-sectional view of a long bone that has been reamed with the reamer assembly of the present invention;

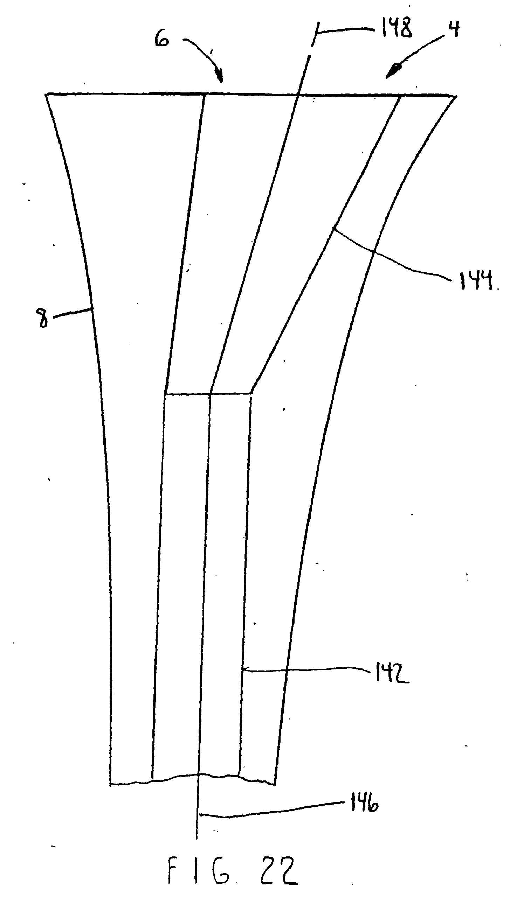

FIG. 22 is a cross-sectional view of a long bone that has been reamed with the reamer assembly of the present invention with the view taken at right angles to that of FIG. 21 showing the proximal reamed portion skewed with respect to the distal reamed portion;

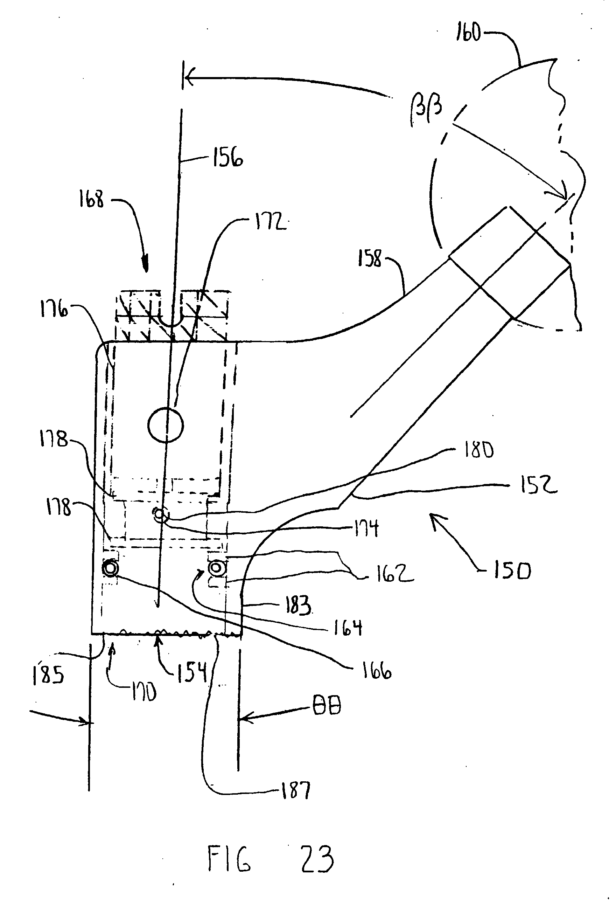

FIG. 23 is a plan view partially in cross-section of a proximal trial for use with the reamer of the reamer assembly of FIG. 1 of the present invention;

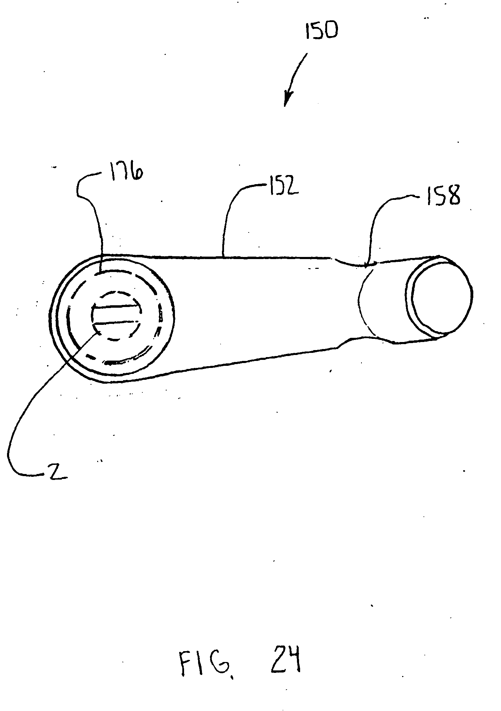

FIG. 24 is a top view of the proximal trial for use with the reamer of the reamer assembly of FIG. 1;

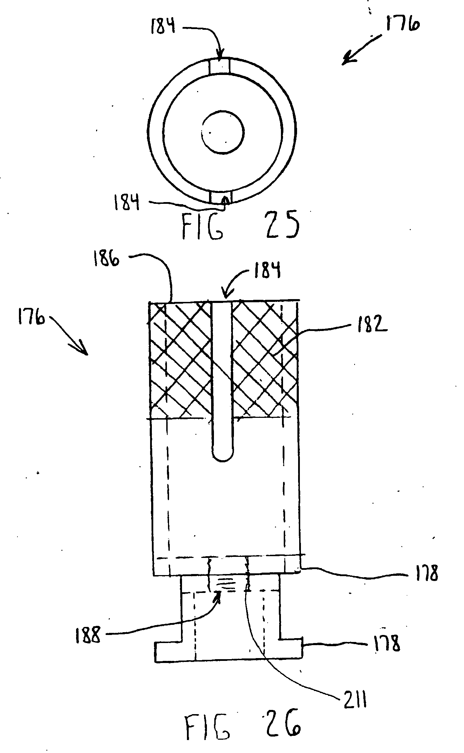

FIG. 25 is a top view of a nut for use with the proximal trial of FIG. 23 and for use with the reamer of the reamer assembly of FIG. 1 of the present invention;
FIG. 26 is a plan view partially in cross-section of the nut of FIG. 25;

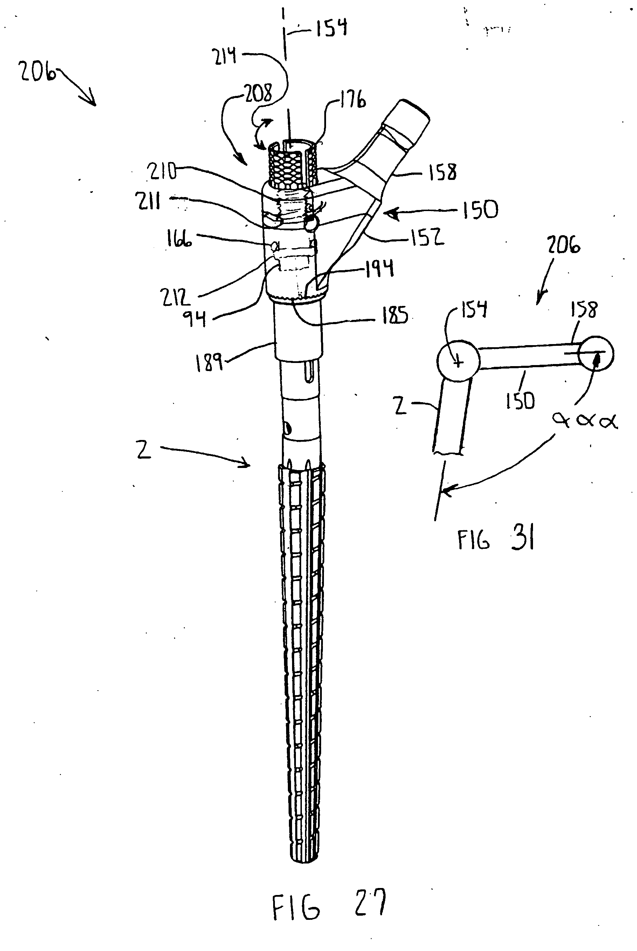

FIG. 27 is a perspective view of the reamer assembly of FIG. 1 with a proximal trial mounted on the reamer assembly;

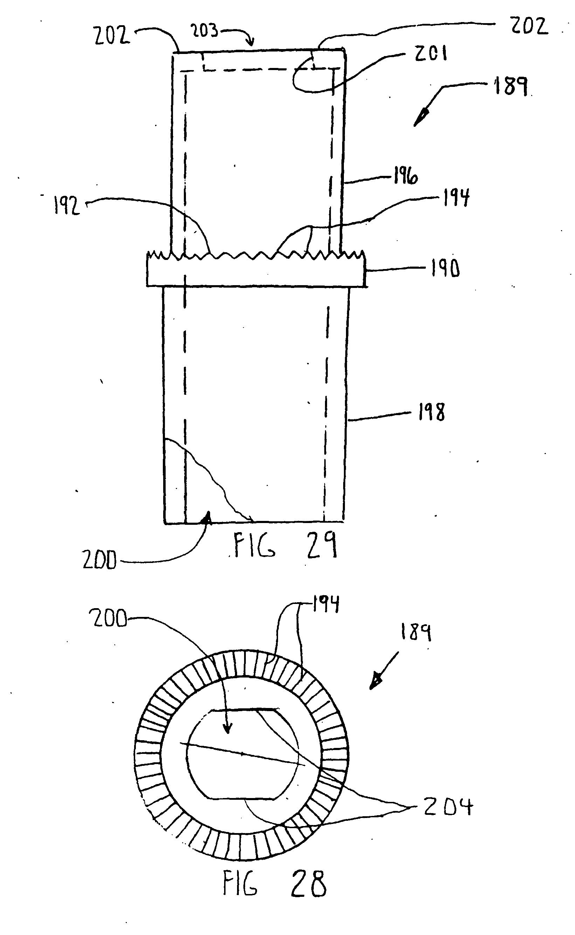

FIG. 28 is a top view of an adapter for use with the proximal trial of FIG. 23 and for use with the reamer of the reamer assembly of FIG. 1 of the present invention;

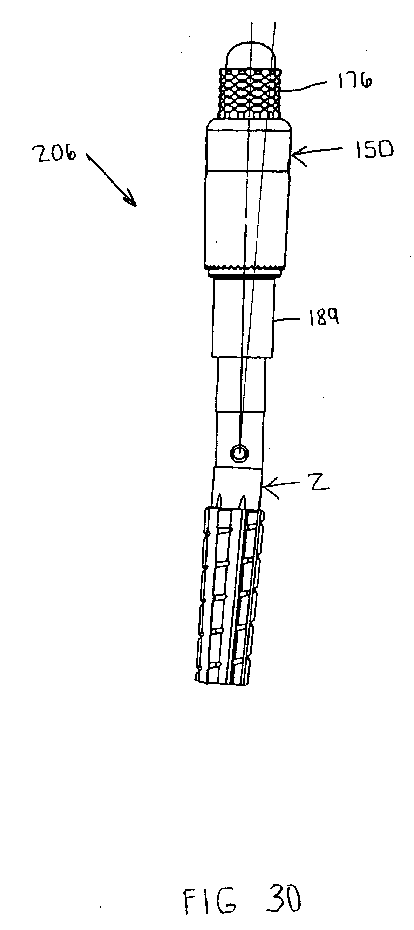

FIG. 29 is a plan view partially in cross-section of the adaptor of FIG. 28;
FIG. 30 is a partial end view of the reamer and proximal trial assembly of FIG. 27;

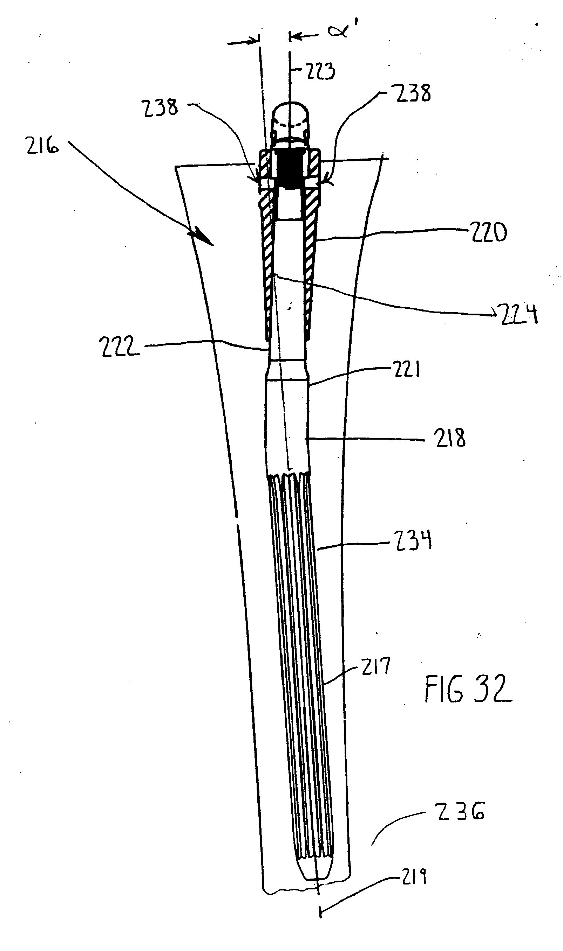

FIG. 31 is a partial top view of the reamer and proximal trial assembly of FIG. 27;
FIG. 32 is an end view partially in cross-section of a prosthesis implanted into the long bone for use in the long bone of FIG. 22;

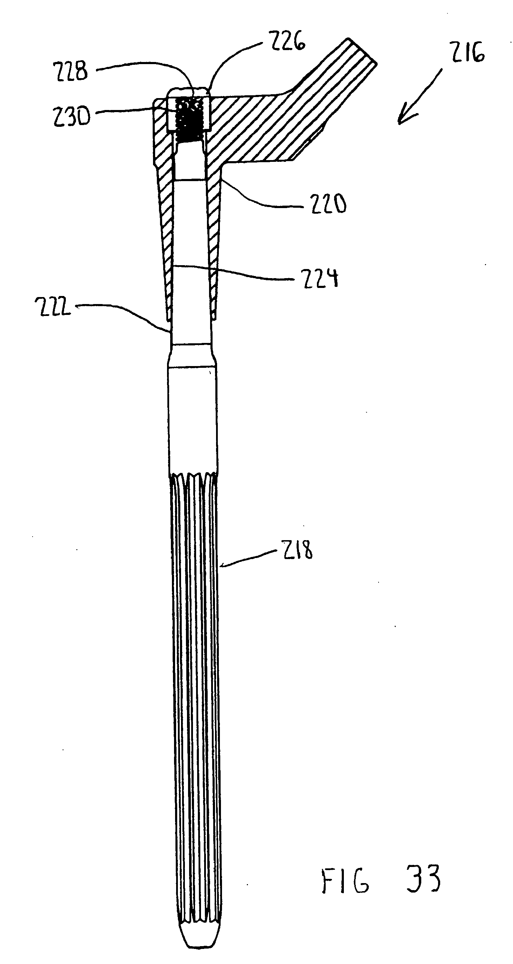

FIG. 33 is a plan view partially in cross-section of the prosthesis of FIG. 32;

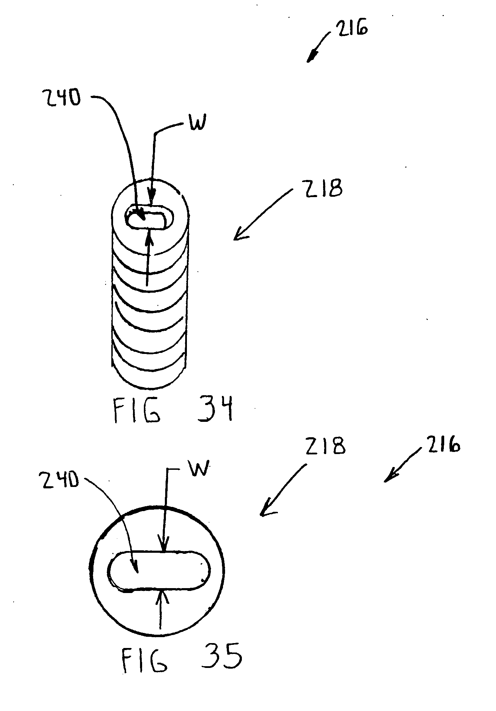

FIG. 34 is a partial perspective view of the threaded end of the prosthesis of FIG. 32;
FIG. 35 is a partial top view of the threaded end of the prosthesis of FIG. 32;

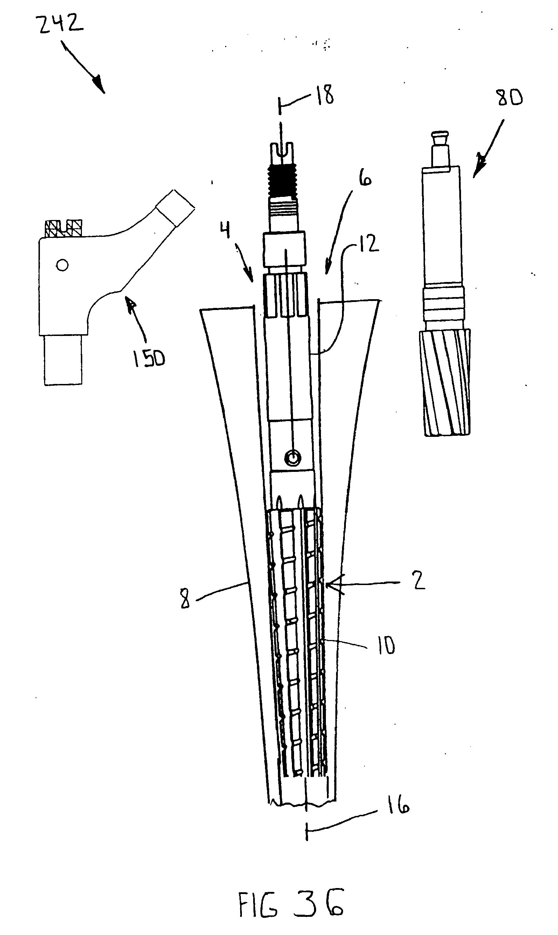

FIG. 36 is a plan view of a kit including the reamer assembly of FIG. 19, the proximal reamer of FIG. 7 and the trial of FIG. 23;

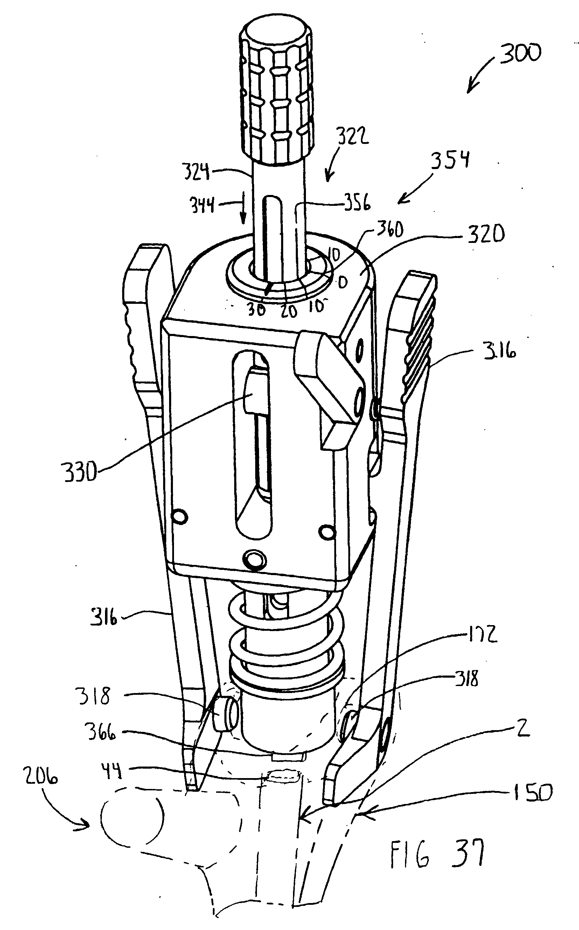

FIG. 37 is a perspective view of an alignment tool shown in use in measuring the alignment of the trial assembly of FIG. 27 and for aligning the prosthesis of FIG. 32 to correspond to the alignment of the trial assembly of FIG. 27 with the trial assembly shown in phantom;

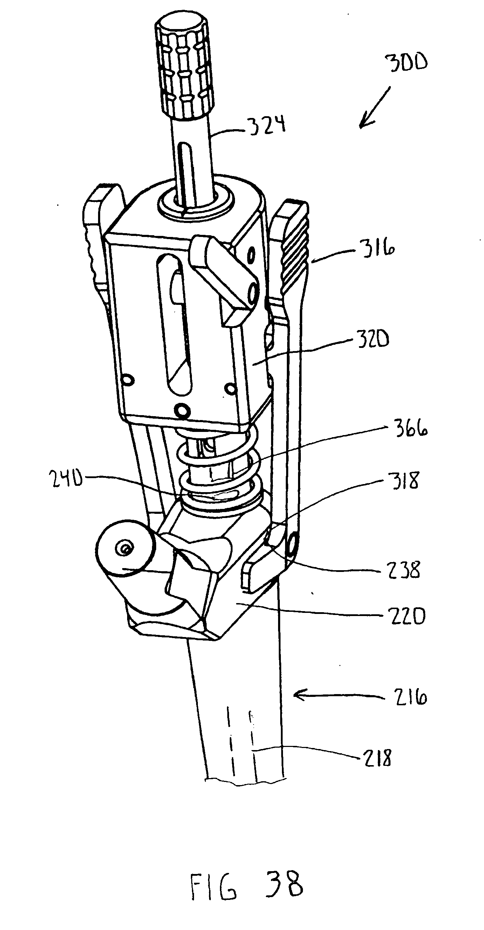

FIG. 38 is a perspective view of the alignment tool shown in use for aligning the prosthesis of FIG. 32 to correspond to the alignment of the trial assembly of FIG. 27 with the trial assembly shown in position on the tool;

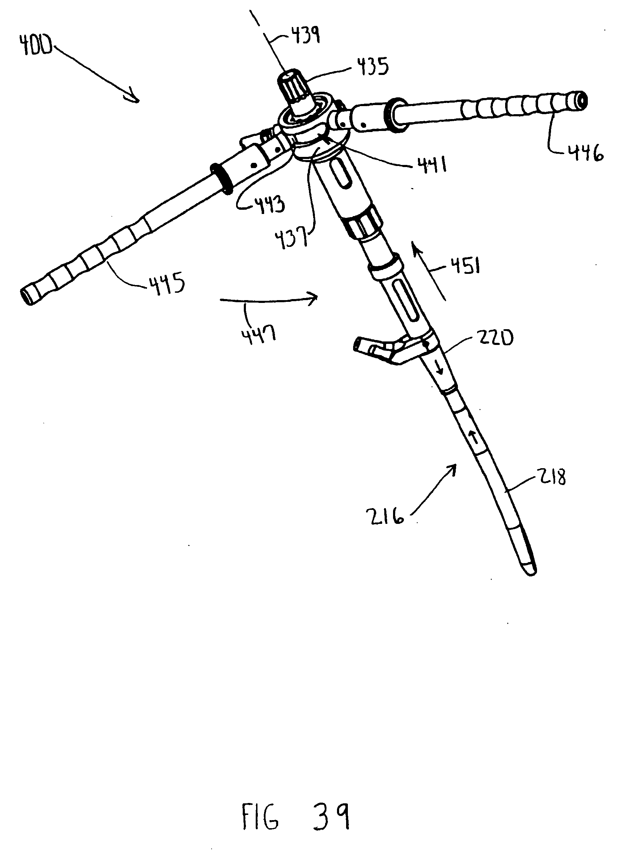

FIG. 39 is a perspective view of an assembly tool shown in use for assembling the prosthesis of FIG. 22;

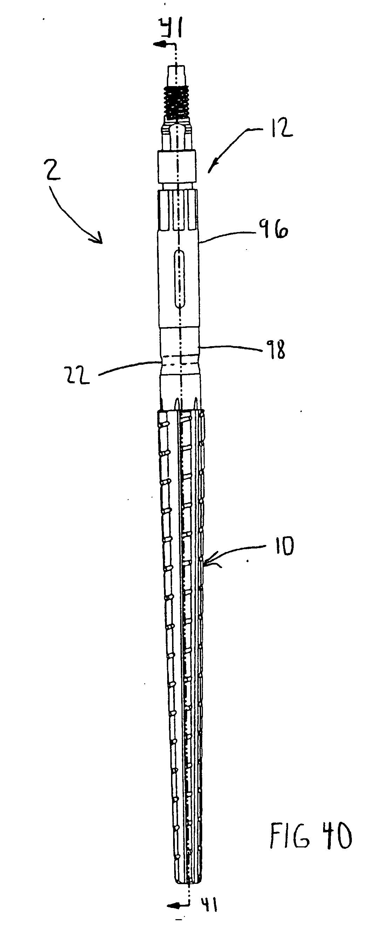

FIG. 40 is a plan view of the distal reamer of the reamer assembly of FIG. 1;

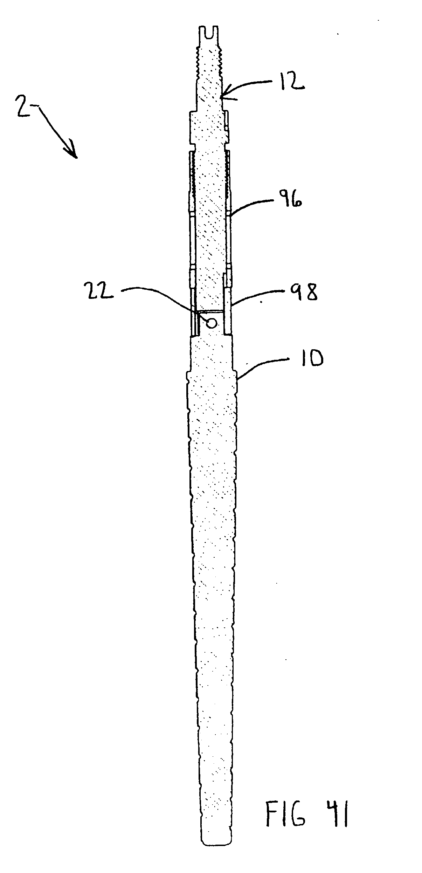

FIG. 41 is a cross sectional view of the reamer of FIG. 40 along the line 41-41 in the direction of the arrows;

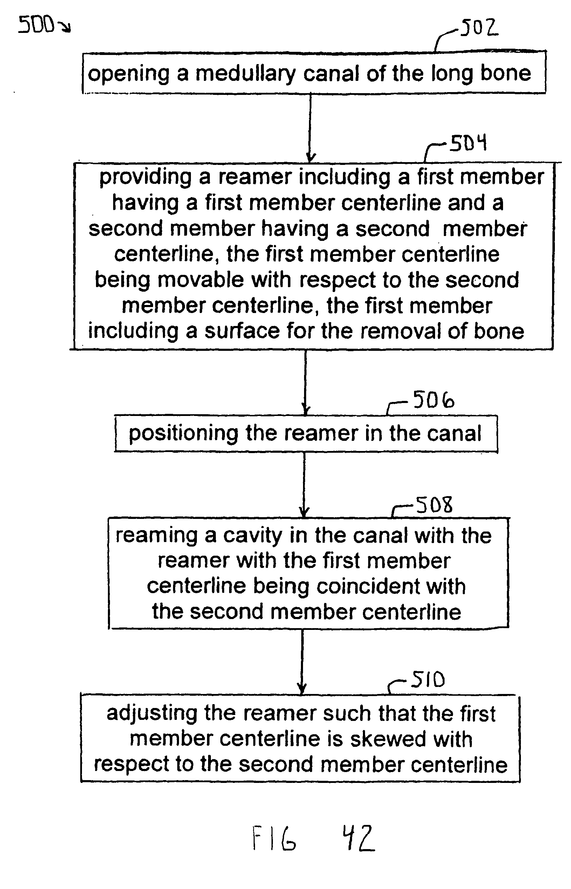

FIG. 42 is a flow chart of the surgical procedure of the present invention utilizing a non-linear reamer;

Referring to the drawings, FIG. 1 shows a reamer 2 which can be used to prepare a cavity 4 in the intramedullary canal 6 of a long bone 8. The reamer 2 includes a first component 10 for preparing the cavity 4 in the canal. The first component 10 includes a portion 14 of the first component 10 for placement at least partially in the canal 4 of the long bone 8. The first component 10 defines a rotational centerline 16 of the first component 10. Reamer 2 also includes a second component 12.

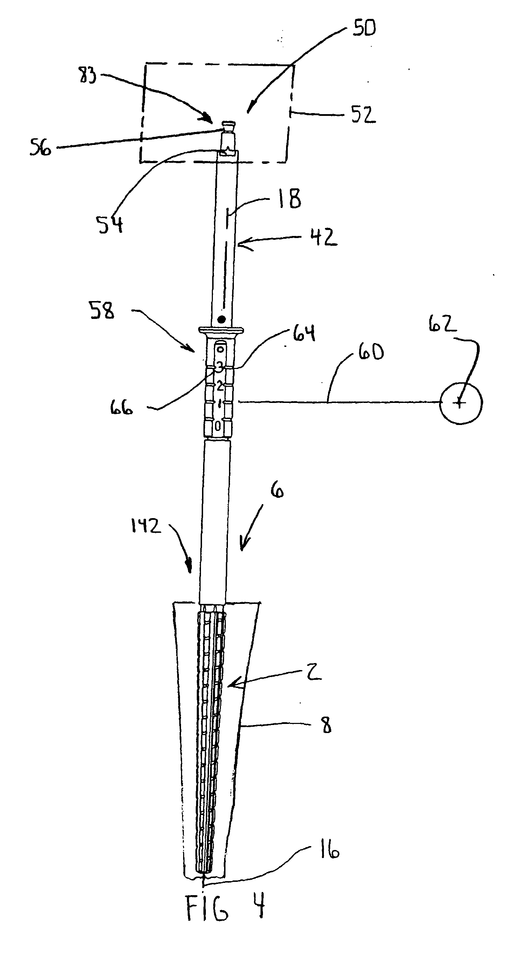

The second component 12 is operably connected to the first component 10. The second component 12 defines a rotational centerline 18 of the second component 12. The rotational centerline 16 of the first component 10 and the rotational centerline 18 of the second component 12 have a first relationship (see FIG. 4) in which the centerlines 16 and 18 are coincident and a second relationship (see FIG. 8) in which the centerlines 16 and 18 are skewed with respect to each other.

The first component 10 and the second component 12 may be operably connected to each other in any way possible to provide for the first relationship and the second relationship. First component 10 and the second component 12 may, as shown in FIG. 1, for example, be pivotally connected. For example, the first component and the second component may be operably connected by a joint 20. The joint 20 is utilized to operably connect the first component 10 to the second component 12. The joint 20 is adapted to provide the first relationship in which the centerlines 16 and 18 are coincident and the second relationship wherein the centerlines 16 and 18 are skewed with respect to each other.

The joint 20 may include, for example, a pin 22, for example, the first component 10 may be rotatably connected to the pin 22 and the second component 12 may likewise be rotatably connected to the pin 22. The first component 10 may include a first component opening 24 for receiving the pin, and the second component 12 may include a second component opening 26 for likewise receiving the pin 22.

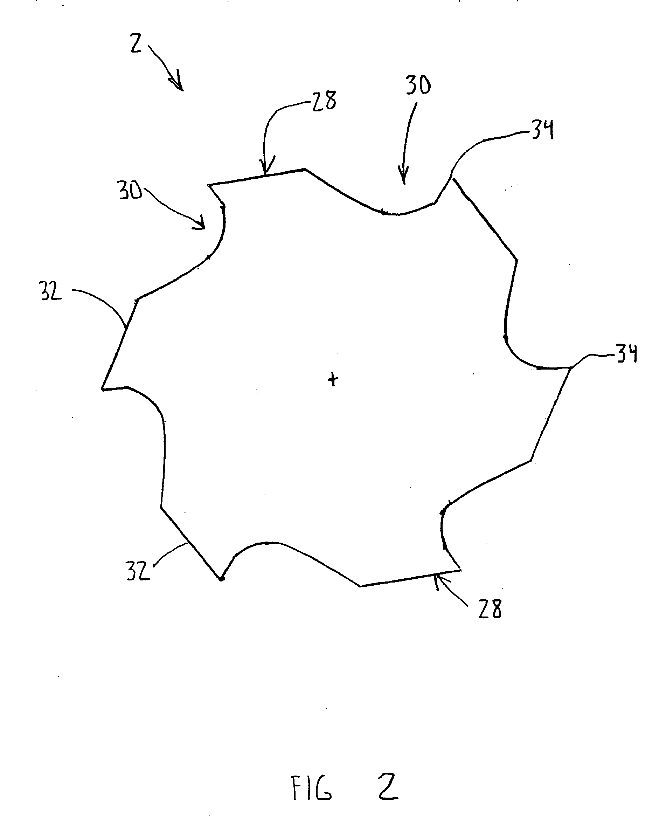

Referring to FIG. 1 and FIG. 2, the first component 10 of the reamer 2 may have any suitable shape. The portion 14 of the first component 10 may have any suitable shape capable of preparing the cavity 4 in the canal 6. The portion 14 may include a plurality of flutes 28. The flutes 28 are separated by reliefs 30. Flutes 28 include land 32 which form cutting edges 34. The portion 14 may be cylindrical or as shown in FIG. 1 may be tapered. If tapered, the portion 14 defines an included angle α. To further assist in bone removal, the portion 14 may include a spiral groove 36.

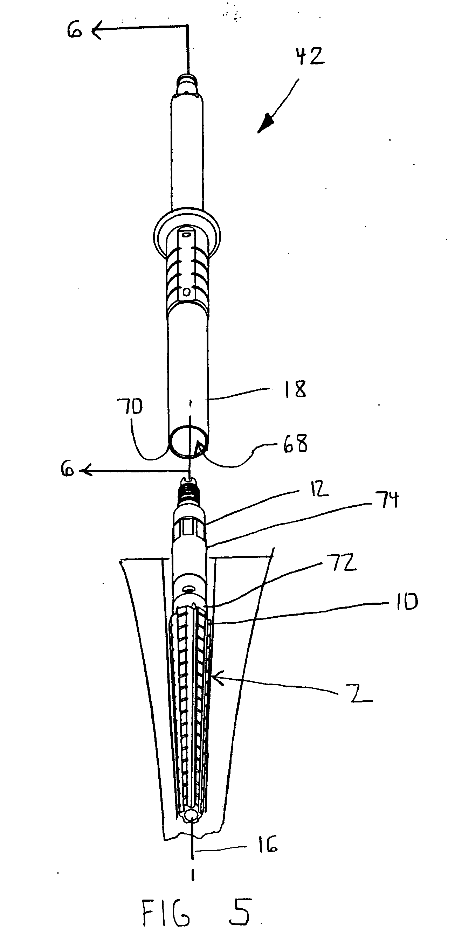

The component 12 may include a portion 38 thereof having a drive connecter 40. Drive connection 40 is used to connect the reamer 2 to driver 42 (See FIG. 5). Drive connecter 40 may have any suitable shape or configuration capable of providing a drive connection to the driver 42.

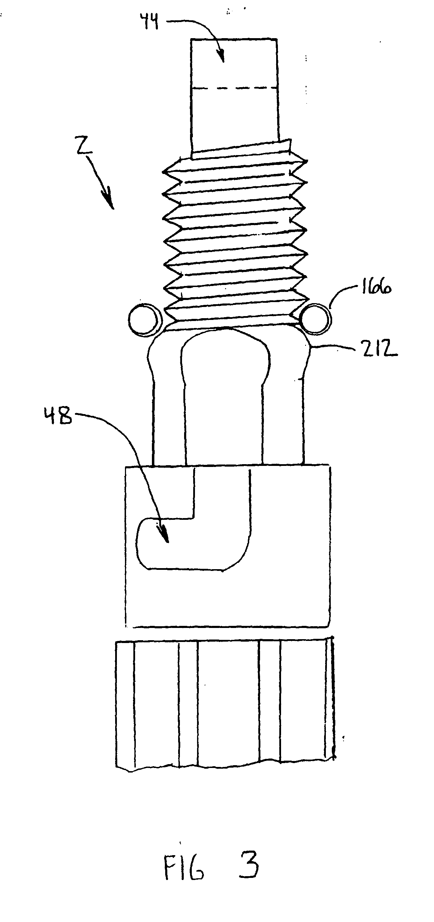

Referring to FIG. 1 and FIG. 3, the drive connecter 40 is shown in greater detail. For simplicity and as shown in FIG. 1 and FIG. 3, the driver connector 40 includes a drive slot 44 extending from end 46 of the component 12 of the reamer 2.

Connector 40 may further include a second feature to assure that the drive slot 44 stays engaged with driver 42. For example and as shown in FIG. 3, the driver connector 40 further includes a bayonet or J-channel 48 for cooperation with the driver 42.

Referring now to FIG. 4, the driver 42 is shown engaged onto distal reamer 2. The driver 42 is placed on the reamer 42 to provide a simple and standard connection for assisting in rotating the reamer 2 and to extend the reamer 2 beyond the canal 6 so that the reamer 2 may be easily rotated outside of the wound. As shown in FIG. 4, the driver 42 includes a drive adaptor 50 that operably connects the driver 42 to a driving device 52 (shown in phantom).

The drive adaptor 50 may be any device capable of rotating the driver 42. For example, the driving device may be a hand tool to be used by the surgeon to perform the operation. Conversely, the driving device 52 may be a power tool, for example, an air driven or electric driven power tool.

The drive adaptor 50 may have any suitable shape and may be, as shown in FIG. 4, in the form of a Hudson connector. The drive adaptor 50 may include flats 54 and an inverted cone 56.

As shown in FIG. 4, the driver 42 may include indicia 58 formed on the driver 42. The indicia 58 may be used to assist in determining the proper depth of the distal reamer 2 during the reaming operation. The indicia 58 will preferably be used in conjunction with a visual reference along visualization line 60 with, for example, a bony landmark on the patient, for example, head centerline 62. The surgeon aligns the head centerline 62 with the indicia 58 to determine the proper depth of the reamer 2. The indicia 58 may be in the form of marks or lines 64. Numbers or letters 66 may be placed adjacent the lines 64 to refer to a particular trial or prosthesis which should be used when the line 64 corresponds to the visualization line 60.

Referring now to FIG. 5, the driver 42 is shown disassembled from the distal reamer 2. For simplicity and as shown in FIG. 5 and FIG. 4, the driver 42 serves to stiffen or to align the first component 10 with the second component 12 such that the first rotational centerline 16 and the second rotational centerline 18 are coincident. Such alignment may be simply performed by providing the driver 42 a cylindrical bore or opening 68 extending inwardly from distal end 70 of the driver 42. The bore 68 is preferably matingly fitted to cylindrical periphery 72 of the first component 10 and matingly fitted to the cylindrical periphery 74 of the second component 12.

The reamer 2 is installed into the driver 42 with the second component 12 being positioned in bore 68 of the driver 42. The outer periphery 72 of the first portion 10 is then placed in bore 68. In this assembled condition, as shown in FIG. 4, the rotational centerline 16 of the first component 10 and the rotational centerline 18 of the second component 12 are coincident.

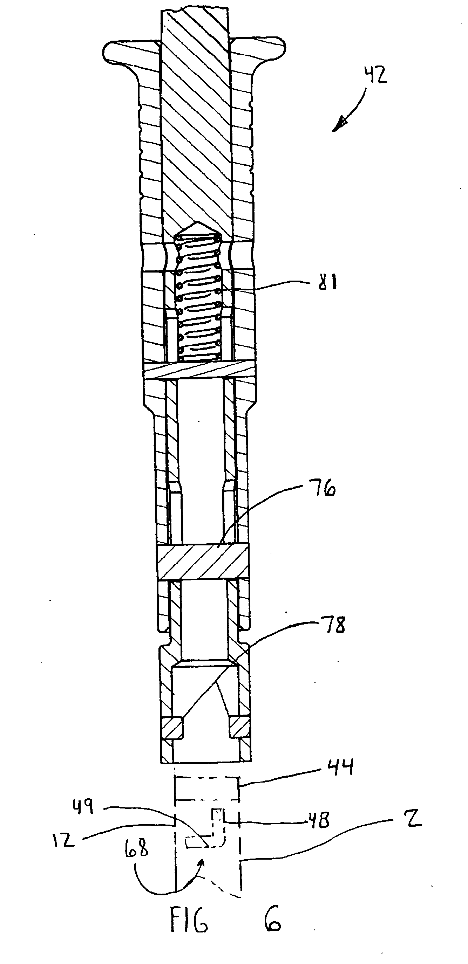

Referring now to FIG. 6, the driver 42 is shown in greater detail. It should be appreciated that any driver capable of securing the reamer and rotating it may be used. The driver 42 as shown in FIG. 6 may include a transverse cross drive pin 76 extending transversely across opening 68 of the driver 42. The driver 42 may also include opposed internal pins 78 extending inwardly into opening 68 of the driver 42. The internal pins 78 cooperate with the bayonet or J-channel 48 formed in the reamer 2. A spring 81 is located in the driver 42 to urge the transverse cross drive pin 76 into engagement with the reamer 2.

To install the reamer 2 into the driver 42, the second component 12 of the reamer 2 is inserted into the opening 68 of the driver 42 until the bayonet or J-channel 48 of the reamer 2 engages internal pins 78. Shoulder 49 of the J-channel 48 of the reamer 2 stops the advancement of the reamer 2 with respect into the driver 42 and reamer 2 is then rotated with respect to the driver 42 until the driver slot 44 is engaged into the transverse cross drive pin 76 of the driver 42, thereby securing the reamer 2 into the driver 42.

Referring now to FIG. 7, a proximal reamer 80 is shown. The proximal reamer 80 is used to provide clearance for the proximal portion of the trials and implants for use in the present invention. Proximal reamer 80 may be any reamer capable of providing clearance for the proximal reamer and proximal trials, and as shown in FIG. 7 may include a cutting portion 82 and a shank 84. The cutting portion 82 is used to prepare the clearance for the trials and implants. The cutting portion may have any suitable shape or configuration for providing the clearance.

The cutting portion 82 may include flutes 86 for cutting bone and other tissue. A central opening 88 may be formed in the reamer 80 extending inwardly from distal end 90 of the cutting portion 82 of the reamer 80. The proximal reamer 80 may further include a driver adapter 92 extending outwardly from the shank 84. The driver adapter 92 may be any drive adapter capable of cooperating with a driving mechanism. The drive adapter 92 may be similar to the driver adapter 50 of the driver 42 of FIG. 4 and may thus be in the form of a Hudson adapter.

Referring to FIG. 5 and FIG. 4, it should be appreciated that for the reamer 2 to fit into the opening 68 of the driver 42, the first component 10 and the second component 12 may freely pivot during entry of the reamer into the opening 68 of the driver 42. Thus, the reamer 2 may be adapted to have a condition in which the first component 10 freely pivots with respect to the second component 12.

Such a free pivoting operation may be accomplished in many different ways. For example and as shown in FIG. 8, the reamer 2 is shown in a configuration in which the second component 12 may freely pivot with respect to the first component 10. As shown in FIG. 8, the second component 12 as shown is modular or consists of several components. For example and as shown in FIG. 8, the second component 12 includes a shaft 94. The shaft 94 is connected to the first component 10 by means of the pin 22. The second component 12 further includes a sleeve 96 which is slidably fitted over the shaft 94 and threadably engaged thereto. The second component 12 further includes a tubular wedge 98 that is slidably fitted over the shaft 94 and is positioned between the sleeve 96 and the second component 12.

As shown in FIG. 8, when the sleeve 96 is threadably positioned into contact with hub 100 of the shaft 94, the tubular wedge 98 may move axially between the sleeve 96 and the shoulder 102 of the second component 12. Since the tubular wedge 98 is free to move axially, the first component 12 is permitted to pivot with respect to the second component 12, thus permitting the reamer 2 to be positioned in the opening 68 of the driver 42 (see FIG. 4).

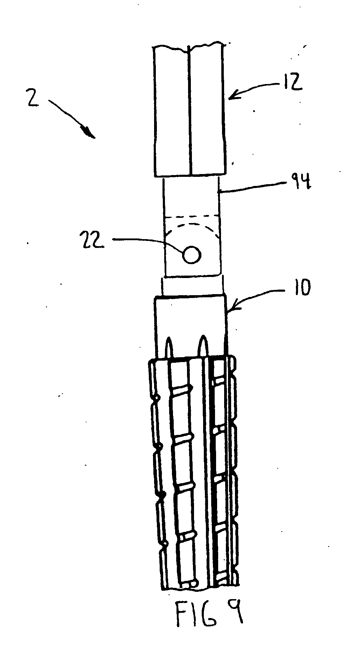

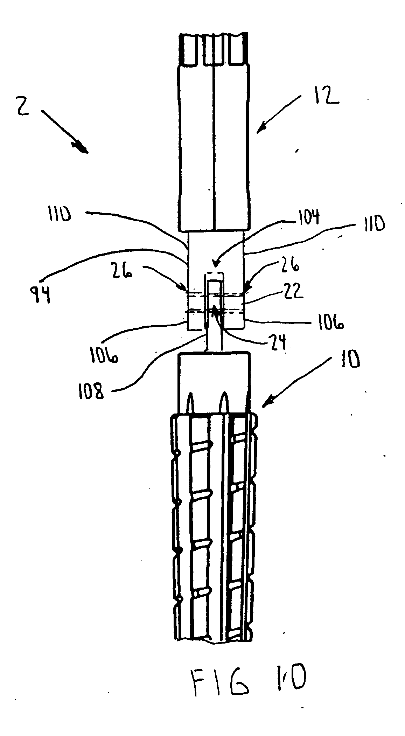

Referring now to FIGS. 9 and 10, the reamer 2 is shown with the tubular wedge 98 removed. As shown in FIGS. 9 and 10, the proximal end of shaft 94 includes a longitudinally elongated slot 104. Slot 104 forms tines 106 positioned between the slot 104. A tang 108 extends proximally from the first component 10. The opening 24 is formed in the tang 108 to receive pin 22. Similarly, openings 26 are formed in tines 106 of the shaft 94 for receiving the pin 22. Flats 110 are formed on the shaft 94 for orienting the tubular wedge 98.

Referring now to FIGS. 11, 12 and 13, the tubular wedge 98 is shown in greater detail. The tubular wedge 98 includes a central opening 112 for slidably fitting over the shaft 94 of FIG. 10. The tubular wedge 98 includes a distal face 114 which is perpendicular to the opening 112 and an opposed skewed proximal face 116 which forms an included angle β between the skewed proximal face 116 and the distal face 114. As one can readily see, the angle β roughly equals angle θ running between first component centerline 16 and second component centerline 18 (see FIG. 14).

The tubular wedge 98 further includes parallel spaced apart flats 118 formed in the opening 112 of the wedge 98. The flats 118 are matingly fitted to the flats 110 on the shaft 94 (see FIG. 10) to angularly orient the wedge 98 with the shaft 94. Pin head installation holes 120 are transversely positioned through the tubular wedge 98 to provide access for the assembly of the pins 22 into the shaft 94 (see FIG. 10).

Referring now to FIG. 14, reamer 2 is shown in the locked position with the first component centerline 16 skewed with respect to the second component centerline 18 by an included angle θ, which is generally similar to the angle β formed by the tubular wedge 98. As the locking sleeve 96 is rotated in the direction of arrow 122 with respect to the shaft 94, the locking sleeve 96 transverses along the second component centerline 18 in the direction of arrow 124, until the sleeve 96 is in contact with the skewed proximal face 116 of the wedge 98.

The locking plate 96 is continued to be rotated in the direction of arrow 122 causing the locking sleeve 96 to continue to move in the direction of arrow 124 until the distal face 114 of the tubular wedge 98 seats against shoulder 102 of the first component 10 of the reamer 2.

The locking sleeve then is torqued into a locked position. Slots 126 may be formed on the locking sleeve 96 to assist in properly torquing the sleeve 96 with respect to the shaft 94.

Referring now to FIGS. 15 and 16, the wrench 128 is shown for use in locking the reamer 2 of FIG. 14. The wrench 128 may have any suitable configuration and may, as shown in FIGS. 15 and 16, include a body 130 which may include knurls 132 for proper gripping. The wrench 128 may include an opening 134 extending inwardly from end 136 of the body 130. Flats 138 may be formed in the opening 134. The flats 138 are preferably matingly fitted to the slots 126 of the locking sleeve 96 of the reamer 2 (see FIG. 14). Rotating the wrench 128 when in position on the reamer 2 may be used to properly secure and lock the reamer 2.

Referring now to FIG. 17, the wrench 128 is shown in position as it is being assembled onto the reamer 2 to torque the locking sleeve 96 to the shaft 94 of the reamer 2.

Referring now to FIG. 18, the wrench 128 is shown with the flats 138 of the wrench 128 fully engaged against the slots 126 of the locking sleeve 96 of the reamer 2.

Referring now to FIG. 19, another embodiment of the present invention is shown as reamer assembly 140. Reamer assembly 140 is utilized for preparing the cavity 4 in the intramedullary canal 6 of the long bone or femur 8. The reamer assembly 140 includes first reamer in the form of, for example, distal reamer 2 as well as second reamer 80 in the form of, for example, a proximal reamer. The first reamer 2 includes a first portion or component 10 for preparation of the cavity 4 in the canal 6. The first portion 10 defines the rotational centerline 16 of the first portion 10. The reamer 2 further includes a second portion in the form of, for example, second component 12 operably connected to the first portion 10. The second portion 12 defines the second portion rotational centerline 18.

The rotational centerline 16 of the first portion 10 and the rotational centerline 18 of the second portion 12 have a first relationship in which the centerlines 16 and 18 are coincident and a second relationship in which the centerlines 16 and 18 are skewed with respect to each other. The second reamer 80 is slidably fitted over at least a portion of the first reamer 2. For example, the second reamer 80 may include the opening 88 into which the second component 12 of the first reamer 2 slidably fits.

Referring now to FIG. 20, after the proximal reamer 2 has properly reamed the distal portion of the femur 8, the driver 42 (see FIG. 5) is separated from the distal reamer 2. Then wrench 128 is utilized to tighten the locking sleeve 96 to the shaft 4 of the distal reamer 2 thereby locking the distal reamer 2 into the articulated skewed position (see FIG. 17). It should be appreciated that the surgeon must orient the skewed position of the distal reamer 2 with respect to the femoral anatomy and the patient's body.

Referring to FIG. 20, when the distal reamer 2 has been properly tightened, the wrench 128 is removed from the distal reamer 2 and the proximal reamer 80 is positioned over the second component 12 of the distal reamer 2. The driving device 52 in the form of either a power tool or a hand tool is attached to the drive adapter 92.

The driving device 52 thus rotates the proximal reamer 80 to prepare the proximal portion of the cavity 4 for receiving the proximal trial or the proximal implant at the correct angle according to the centerlines 16 and 18.

Referring now to FIGS. 21 and 22, anterior/posterior and lateral views, respectively, the prepared cavity 4 in the intramedullary canal 6 of the long bone 8 is shown in greater detail. As shown in FIGS. 1 and 4, distal reamer 2 is used to prepare distal reamed portion 142 of the cavity 4, while the proximal reamer 80 (see FIG. 20) is used to prepare the proximal portion 144 of the cavity 4. The distal reamed portion 142 is defined by a centerline 146 while the proximal reamed portion 144 is defined by a centerline 148.

It should be readily observed that FIGS. 21 and 22 are shown as a view through the plane defining the distal rotational centerline 146 and the proximal rotational centerline 148. Thus, as shown in FIG. 22, the centerlines 146 and 148 are skewed with respect to each other. Since FIG. 21 is shown as a cross-section normal to that of the cross-section of FIG. 22, the centerlines 146 and 148 of FIG. 21 appear parallel.

Referring now to FIGS. 23 and 24, a proximal trial 150 is shown for use with the reamer 2 of the present invention. Proximal trial 150 may be made of any suitable durable material, for example, a plastic or a metal. If made of a metal, the proximal trial 150 may be made of any readily machinable material that may be sterilized using an autoclave or other sterilization techniques.

The reamer 2, the driver 42 and the reamer 80 may be made of any suitable durable material that may be sterilized using standard sterilization techniques such as an autoclave. Preferably the reamer 2, driver 42 and reamer 80 may be made of a durable material, for example a metal.

Proximal trial 150 may include a body 152 defining a central opening 154 concentric with longitudinal centerline 156 of the proximal trial 150. A neck 158 extends outwardly from the body 152 at an angle ββ or neck angle ββ from the longitudinal centerline 156 of, for example, 30 to 80°. A head 160 (shown in phantom) may be positioned on the neck 158.

A pair of spaced apart parallel internal flanges 162 may extend inwardly from the body 152 into the central opening 154. Internal flanges 162 define a groove 164 for containing spring 166. The internal flanges 162 divide the central opening 154 into a proximal opening portion 168 and a distal opening portion 170. Opposed assembly and locking holes 172 may be formed through the body 152 in alignment with the centre opening 154. Opposed locking pin holes 174, likewise, may be formed in the body 152 in alignment with the central opening 154.

A nut 176 (shown in phantom) may be positioned in the proximal opening portion 168 of the central opening 154 of the body 152 of the proximal trial 150. Nut 176 may be used to secure the reamer 2 to the proximal trial 150. Nut 176 is slidably fitted to the proximal opening portion 168. Nut 176 may include a pair of spaced apart flanges 178 which cooperate with a pin 180 fixed into the locking pin holes 174 for pinning the nut 176 within the centre opening 154.

Referring now to FIGS. 25 and 26, the nut 176 is shown in greater detail. The nut 176 may include knurls 182 formed on the nut 176 to assist in torquing the nut 176 onto the reamer 2. The nut 176 may further include a pair of spaced apart axial slots 184 extending distally from the proximal end 186 of the nut 176. The nut 176 may further include a central threaded opening 188 for cooperation with the reamer 2.

Referring now to FIG. 27, it should be appreciated that the proximal trial 150 may be directly connected to the reamer 2. It should also be appreciated that in order for the reamer 2 to be connectable to both the driver 42 as well as the proximal trial 150, an adaptor such as adaptor 182 may be used to provide a connection for the proximal trial 150 that may be more conducive for a proximal trial that may also be usable with distal stem trials (not shown). Therefore, as shown in FIG. 27, an adaptor 189 may be used to connect the proximal trial 150 to the reamer 2 and may be pinned to the proximal trial 150.

Referring again to FIGS. 23 and 24, the proximal trial 150 further includes a frustoconical portion 183 formed on the body 150 and being concentric with the longitudinal centerline 156. The frustoconical portion 183 has an included angle θθ preferably similar to the included angle αα of the cutting portion 82 of the proximal reamer 80 (see FIG. 7)and preferably similar to the corresponding geometry implant.

Radial teeth 185 may be formed on distal end 187 of the frustoconical portion 182 of the body 152 of the proximal trial 150. Any number of radial teeth may be used for the radial teeth 185. For example, thirty-six (36) radial teeth may be equally spaced along the end 186. Each of the thirty-six (36) teeth would then represent a 10 degree rotation from the longitudinal centerline 156.

Referring now to FIGS. 28 and 29, the adaptor 189 is shown in greater detail. The adaptor 189 includes a central ring 190. The central ring 190 includes a proximal face 192. The proximal face 192 includes a plurality of spaced-apart radial teeth 194. The radial teeth 194 preferably mate with radial teeth 185 of the proximal trial 150 (see FIG. 23). The adaptor 189 further includes a first tubular portion 196 that extends outwardly from the first face 192 of the central ring 190 of the adaptor 189. A second tubular portion 198 extends in the direction opposed to the first tubular portion 196 portion from the central ring 190. A cylindrical through hole 200 is formed in the first tubular portion 196, the central ring 190 and the second tubular portion 198. An internal flange 201 extends inwardly from the cylindrical through hole 203 at the proximal end 202 of the adaptor 189. The flange 201 forms opposed parallel flats 204.

Referring again to FIG. 27 and to FIG. 30, the adaptor 189 is shown in position on the reamer 2. The proximal trial 150 is shown in position on the reamer 2 and in position against the adaptor 189. Nut 176 is shown assembled into the proximal trial 150 and into the distal reamer 2. The reamer 2, adaptor 189, proximal trial 150 and nut 176 combine to form a trial reamer assembly 206.

Referring now FIG. 31, it should be appreciated that since the reamer 2 is skewed or non-linear and the proximal trial 150 includes the neck 158 extending from the centerline 154 of the trial 150, during assembly the neck 158 and the reamer 2 need to be aligned with respect to the centerline or trial axis 154. As shown in FIG. 31, the reamer 2 and the neck 158 form an angle αα with respect to the trial centerline 154. This angle αα must be set during the assembly of proximal trial 150 onto the reamer 2.

Referring now to FIGS. 17 and 27, and according to an aspect of the present invention, the reamer trial assembly 206 may include a angular orientation feature 208 which may be use to precisely vary the angle ααα (see FIG. 31).

Referring to FIG. 17, external threads 210 are shown positioned between the slot 44 and the body 94. The external threads 210 mate with internal threads 211 formed on nut 176. (See FIG. 26).

Referring again to FIG. 27, the orientation feature 208 includes a flange 212 that extends outwardly from the body 94 of the reamer 2. As the reamer 2 is moved into engagement with the proximal body 152, the flange 212 of the reamer 2 snaps over the spring 166 of the proximal body 150 providing a snap-in feature for the reamer trial assembly 206. The nut 176 includes the internal threads 211 which are threadably engaged with external threads 210 on the reamer 2. As the threads 211 and 210 are threadably engaged, the reamer 2 becomes increasingly engaged with the proximal body 150. The threaded engagement is set to provide axial and thus rotational stability between the proximal body 150 and the reamer 2.

The spring 166 and flange 212 thus serve to hold temporarily the proximal body 150 to the reamer 2. In this condition the proximal body 150 may be rotated with respect to the reamer 2 in the direction of arrow 214 to adjust anteversion. The spring 166 mating with the flange 212 urges the radial teeth 185 of the proximal trial into engagement with radial teeth 194 of the adaptor 189. Radial teeth 185 and radial teeth 194 thus ratchet or click as the proximal body 150 is rotated with respect to the reamer 2. If, for example, the teeth 194 include thirty-six (36) spaced apart teeth and the teeth 185 include thirty-six (36) spaced apart teeth, each click or index of the proximal body 150 with respect to the reamer 2 provides for a change in angle αα of 10°. Thus, the orientation feature 208 serves to provide for accurate indexable orientation, for example, anteversion of the proximal body 150 with respect to the reamer 2.

Referring to FIGS. 32, 33, 34 and 35, a prosthesis 216 is shown for use with the reamer and trial of the present invention. The prosthesis 216 includes a distal stem 218 that matingly engages a proximal body 220. The distal stem 218 and proximal body 220 may be matingly engaged in any suitable manner such as, for example, shown in FIGS. 32 and 33 by an external taper 222 formed on the distal stem 218 which mates with an internal taper 224 formed on the proximal body 220.

The distal stem 216 may, as shown in FIG. 32, have a distal portion 217, have a longitudinal centerline 219, which is skewed with a proximal portion 221 of stem 216, the portion 211 having a longitudinal centerline 223. The centerlines 219 and 223 of portions 217 and 221 form an included α which may be similar to angle α of the reamer 2 of FIG. 1.

Referring now to FIG. 33, while it should be appreciated that the internal taper 224 and the external taper 222 may be sufficient to properly secure the stem to the body, a nut 226 may be used to secure the body 220 to the stem 216. The nut 226 may include internal threads 228 that mate with external threads 230 formed on the distal stem 218.

In order to orient the body 220 to the distal stem 218, the proximal body 220 may have features in the form of, for example, removal and location holes 238 formed through the proximal body 220.

Referring now to FIGS. 34 and 35, the distal stem 218 may include an angular location feature in the form of an elongated slot 240. Elongated slot 240 defines a width W that may be similar to the width SW of slot 44 of the reamer (See FIG. 8).

Referring now to FIG. 36, another embodiment of the present invention is shown as kit 242. The kit 242 is used for preparing the cavity 4 in the intramedullary canal 6 of a long bone 8 for use in performing joint arthroplasty. The long bone 8 may be a femur, a humerus or any suitable long bone. The kit 242 includes the first reamer 2 and the trial 150. The first reamer 2 includes the first portion 10 for preparing the cavity 4 in the canal 6. The first portion 10 defines the rotational centerline 16 of the first portion 10. The kit 242 may also include a drive such as driver 42 of FIG.5.

The first reamer 2 also includes the second portion 12 that is operably connected to the first portion 10. The second portion 12 defines the rotational centerline 18 of the second portion 12. The rotational centerline 16 of the first portion 10 and the rotational centerline 18 of the second portion 12 have a first relationship in which the centerlines are coincident, and a second relationship in which the centerlines are skewed with respect to each other. Trial 150 is utilized for assisting in performing a trial reduction. The trial 150 is operably associated with the first reamer 2.

The kit 242 may further include the second reamer 80. The second reamer 80 is adapted to be slidably fittable over at least a section of the second portion 12 of the first reamer 2.

Referring now to FIG. 37, an alignment device 300 is shown in position on the reamer trial assembly 206. The alignment device 300 is utilized to first obtain an orientation of the trial 150 with respect to the reamer 2 and to be able to transfer that orientation onto the prosthesis.

The alignment device 300 may be any device capable of transferring this relationship. For example, and as shown in FIG. 37, the alignment device 300 includes a body 320 which includes a central opening 322 to which rod 324 slidably fits and moves in the direction of arrow 344. Arms 316 are pivotally secured to the body 320. Pins 318 extend inwardly from the arms 316.

Tip 366 located on the distal end of rod 324 is matingly fitted to engage with slot 44 of the reamer 2 of the reamer trial assembly 206. Further the pins 318 engage rotation holes 172 formed on the trial 150 of the reamer trial assembly 206. With the engagement of the slot 44 to the tip 366 and the location holes 172 with the pins 318, the rod 324 is oriented with respect to the body 320 in a fashion similar to the orientation of the reamer 2 to the trial 150 of the reamer trial assembly 206.

The relationship of the body 320 with respect to the rod 324 may be recorded by use of, for example, indicia 354. The indicia 354 may be in the form of, for example, lines 360 on body 320 which may align with a line 356 formed on the rod 324. In addition, the alignment of the body 320 to the rod 324, may be secured by means of, for example, a locking mechanism 330 used to rotatably lock the body 320 to the rod 324.

Referring now to FIG. 38, the alignment device, is shown in engagement with the prosthesis 216. To rotatably align the proximal body 220 to the distal stem 218, the tip 366 of the rod 324 of the alignment device 300 engages slot 240 formed on the stem 218 of the prosthesis 216. Similarly, the pins 318 mounted on the arms 316 secured to the body 320 of the alignment device 300 engages the holes 238 of the body 220 of the prosthesis 216. Thus the alignment device 300 rotatably aligns the proximal body 220 to the distal stem 216 in a similar fashion to the orientation of the reamer trial assembly 206 of FIG. 37.

It should be appreciated that a slight tap between the body 220 and the stem 218 may temporarily angularly secure the body 220 to the stem 218 of the prosthesis 216 until the device may be more permanently secured.

Referring now to FIG. 39, an assembly device 400 is shown for use in assembling the prosthesis 216. Any tool capable of joining the distal stem 218 with the proximal body 220 may be used. For example and as shown in FIG. 39, the assembly device 400 may include a second member 435 in the form of, for example, an elongated shaft. The second member 435 is operably associated with the distal stem 218 and may, for example, be threadably connected to the distal stem 218.

The assembly device 400 may further include a first member 437 operably associated with the second member 435. The first member 437 may in the form of, for example, a hollow cylinder that slidably fits over the second member 435. The first member 437 may include a portion which stops against the proximal body 220. The second member 435 may include an actuating arm 445 which includes a pin 443 which is matingly slidably fitted to a helical elongated slot 441 formed in the first member 437.

As the actuating arm 445 is rotated in the direction of arrow 447 about centerline 439 toward the restraining arm 446, the second member 435 is urged in the direction of arrow 451 causing the distal stem 218 to move in the direction of arrow 451 with respect to the proximal body 220, thereby securing the prosthesis 216.

Referring now to FIGS. 40 and 41, the reamer 2 is shown in greater detail. The reamer 2 includes the first portion 10 that is pivotally connected to the second portion 12 by, for example, the pin 22. A tubular wedge 98 is fitted over a first portion 10 and the second portion 12 adjacent the pin 22. The locking sleeve 96 is slidably fitted over the second portion 12 and threadably engages the second portion 12 to securely wedge the tubular wedge 98 between the first portion 10 and the locking sleeve 96, thereby locking the reamer 2 in its bent or articulated position.

Referring now to FIG. 42, another embodiment of the present invention is shown as surgical technique or method 500. The method 500 includes a first step 502 of opening a medullary canal of the long bone. The method 500 further includes a second step 504 of providing a reamer including a first member having a first member centerline and a second member having a second member centerline, the first member centerline being movable with respect to the second member centerline, the first member including a surface for removal of bone. The method 500 further includes a third step 506 of positioning the reamer in the canal and a fourth step 508 of reaming a cavity in the canal with the reamer with the first member centerline being coincident with the second member centerline. The method 500 further includes a fifth step 510 of adjusting the reamer such that the first reamer centerline is skewed with respect to the second member centerline.

## Claims

1. A reamer for preparing a cavity in the intramedullary canal of a long bone, which comprises:
a distal component (10) for preparation of the cavity in the canal, the distal component including a first reamer portion (14) thereof for placement at least partially in the cavity of the long bone, the distal component defining a rotational centerline (16) thereof,
a proximal component (12) on which a second reamer portion or a trial component can be mounted, which is connected to the distal component by means of a hinge (20), the proximal component defining a rotational centerline (18) thereof, the rotational centerline of the distal component and the rotational centerline of the proximal component having a first relationship in which the centerlines are coincident and a second relationship in which the centerlines are skewed with respect to each other, and
a locking arrangement (96, 98) by which the angular relationship between the distal and proximal components can be locked against movement from the second relationship towards the first relationship.

2. A reamer as claimed in claim 1 in which the distal component includes a portion (14) thereof having a tapered external periphery; and in which the proximal component includes a portion (38) thereof having a drive connection (40).

3. A reamer as claimed in claim 1, in which the locking arrangement comprises at least one of a wedge (98) and a pin (22).

4. A reamer assembly which includes a second reamer portion (80) which can be fitted over at least a portion of the second component by sliding.

5. A kit for preparing a cavity in the intramedullary canal of a long bone for use in performing joint arthroplasty, which comprises a reamer (2) as claimed in claim 1, and a trial (150) for assisting in performing a trial reduction which can be fitted to the reamer.

## Patentansprüche

1. Reibahle zur Herstellung eines Hohlraumes in dem intramedullären Kanal eines langen Knochens, die folgendes umfasst:
eine distale Komponente (10) zur Herstellung des Hohlraumes in dem Kanal, wobei die distale Komponente einen ersten Reibahlenabschnitt (14) von dieser zur mindestens teilweisen Platzierung in dem Hohlraum des langen Knochens umfasst, und die distale Komponente eine Rotationsmittelachse (16) festlegt,
eine proximale Komponente (12), auf der ein zweiter Reibahlenabschnitt oder eine Probekomponente angebracht werden kann, die mit der distalen Komponente mittels eines Gelenks (20) verbunden ist, wobei die proximale Komponente eine Rotationsmittelachse (18) von dieser festlegt, die Rotationsmittelachse der distalen Komponente und die Rotationsmittelsachse der proximalen Komponente eine erste Relation haben, in der die Mittelachsen übereinstimmen, und eine zweite Relation haben, in der die Mittelachsen in Bezug zueinander schräg verlaufen, und
eine Arretiervorrichtung (96, 98), durch die die Winkelrelation zwischen der distalen und proximalen Komponente gegen die Bewegung aus der zweiten Relation in die erste Relation arretiert werden kann.

2. Reibahle nach Anspruch 1, in der die distale Komponente einen Abschnitt (14) von dieser umfasst, der eine sich verjüngende äußere Peripherie aufweist; und in der die proximale Komponente einen Abschnitt (38) von dieser umfasst, der eine Antriebsverbindung (40) hat.

3. Reibahle nach Anspruch 1, in der die Arretiervorrichtung mindestens einen Keil (98) und einen Stift (22) umfasst.

4. Reibahlenanordnung, die einen zweiten Reibahlenabschnitt (80) umfasst, der mindestens über einen Teil der zweiten Komponente durch Gleiten angepasst werden kann.

5. Kit zur Herstellung eines Hohlraumes in dem intramedullären Kanal eines langen Knochens zur Verwendung bei der Durchführung einer Gelenkarthroplastik, der eine Reibahle (2) nach Anspruch 1 umfasst, und eine Probe (150), die an die Reibahle angepasst werden kann, zur Unterstützung bei der Durchführung eines Probelaufes.

## Revendications

1. Alésoir pour préparer une cavité dans un canal intramédullaire d'un os long, comprenant :
➢ un composant distal (10) pour la préparation de la cavité dans le canal, le composant distal comprenant une première partie formant alésoir (14) destinée à être placée au moins partiellement dans la cavité de l'os long, le composant distal définissant un axe central de rotation (16) de celui-ci,
➢ un composant proximal (12) sur lequel peut être monté une seconde partie formant alésoir ou un composant d'essai, qui est relié au composant distal au moyen d'une articulation (20), le composant proximal définissant un axe central de rotation (18) de celui-ci, l'axe central de rotation du composant distal et l'axe central de rotation du composant proximal ayant une première relation dans laquelle les axes centraux coïncident et une seconde relation dans laquelle les axes centraux sont inclinés l'un par rapport à l'autre, et
➢ un agencement de blocage (96, 98) au moyen duquel la relation angulaire entre les composants distal et proximal peut être bloquée pour empêcher le mouvement de la seconde relation vers la première relation.

2. Alésoir selon la revendication 1, dans lequel le composant distal comprend une partie (14) ayant une périphérie externe conique ; et dans lequel le composant proximal comprend une partie (38) ayant une liaison d'entraînement (40).

3. Alésoir selon la revendication 1, dans lequel l'agencement de blocage comprend au moins un d'une cale (98) et d'un axe (22).

4. Ensemble alésoir qui comprend une seconde partie formant alésoir (80) qui peut être fixée au-dessus d'au moins une partie du second composant par coulissement.

5. Kit permettant de préparer une cavité dans le canal intramédullaire d'un os long, destiné à être utilisé dans la réalisation d'une arthroplastie articulaire, comprenant un alésoir (2) selon la revendication 1, et dispositif d'essai (150) pour aider à la réalisation d'une réduction d'essai qui peut être fixé à l'alésoir.
